(19) Europäisches Patentamt
European Patent Office
Office européen des brevets

(11) EP 3 037 414 A1

(12) EUROPEAN PATENT APPLICATION

(43) Date of publication:
29.06.2016 Bulletin 2016/26

(51) Int Cl.:
*C07D 209/86* (2006.01) *H01L 51/52* (2006.01)

(21) Application number: 14199901.1

(22) Date of filing: 22.12.2014

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: Solvay SA
1120 Bruxelles (BE)

(72) Inventor: Virboul, Morgane
1060 Brussels (BE)

(74) Representative: Dr. Langfinger & Partner
In der Halde 24
67480 Edenkoben (DE)

# (54) Novel compounds for use in organic electronic devices

(57) Compounds of formula (1)

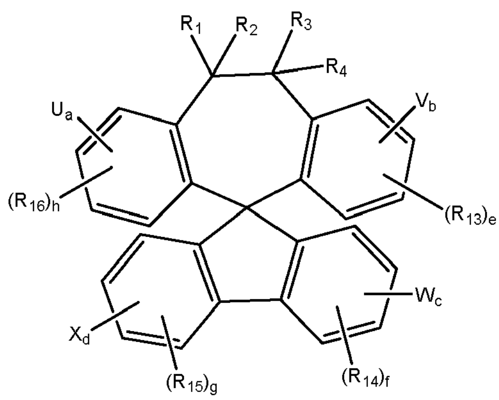
(1)

wherein U, V, W and X, which may be the same or different at each occurrence, represent a substituent group selected from wherein

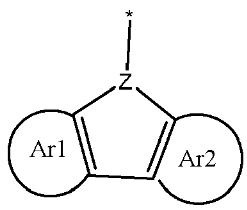
(2)

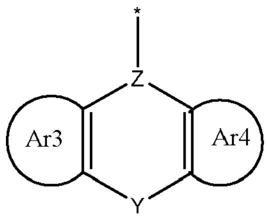
(3)

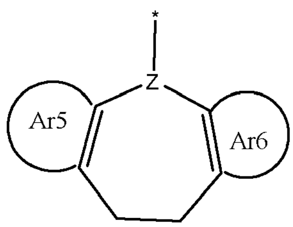
(4)

Z is N or P,
Y is $CR_5R_6$, $NR_7$, $SiR_8R_9$, $PR_{10}$, $P(=O)R_{11}$ or $BR_{12}$

EP 3 037 414 A1

## Description

The invention relates to novel dibenzosuberane-based compounds and to their use in organic electronic devices and to organic electronic devices comprising such compounds.

Organic light-emitting diodes (OLEDs) are an important feature in modern display and lighting technologies, such as, for example, full-color flat displays, flexible displays, and solid-state lighting. Phosphorescent organic light-emitting diodes (PhOLEDs), an important class of OLEDs, are theoretically capable of achieving a 100% internal quantum efficiency by fully harvesting both singlet and triplet excitons. Therefore, PhOLEDs have attracted much attention for their applications in full-color displays and lighting. One promising strategy to obtain highly efficient PhOLEDs is to utilize high triplet energy materials to confine triplet excitons inside an emission layer (EML) in multilayered device structures.

High triplet energy materials are mainly used in EMLs as a host material or in adjacent hole transport layers (HTL) and electron transport layers (ETL). Use of high triplet energy confines triplet excitons inside the EML and suppresses triplet exciton quenching. In multilayered PhOLEDs, the ETL plays an important role in facilitating electron-injection/transport from a cathode while also acting as efficient exciton blocker. It is therefore preferable that the ETL have good electron-transport property, wide energy gap and high triplet energy. A highest occupied molecular orbital (HOMO) level of the electron-transport material is preferably deep enough to block hole carrier leakage and a lowest unoccupied molecular orbital (LUMO) level is preferably low enough to enable efficient electron injection from the cathode. Electron transport materials with high triplet energy preferably exhibit electrochemical, photochemical, and morphological stability.

Various electron-transport materials (ETMs) such as pyridine, phenylpyrimidine, triazine, quinoline, and phosphine oxide (PO) derivatives have been mainly used to achieve high-performance PhOLEDs. Dibenzothiophene-S,S-dioxide and thiophene-S,S-dioxide oligomers and polymers have not been usually viewed as suitable ETMs for PhOLED devices. Although they function as good ETMs for devices with high electron mobilities ($10^{-4}$ - $10^{-3}$ cm2 $V^{-1}$ $s^{-1}$), their low band gap and low triplet energy are in many cases not suitable for efficient PhOLEDs, especially for a blue triplet emitter with high triplet energy.

Korean Patent Application KR2012047038 describes amine derivatives and organoelectroluminiscent devices employing these compounds. There are a number of compounds disclosed comprising a dibenzosuberane unit coupled to a fluorene unit thereby yielding the following core structure

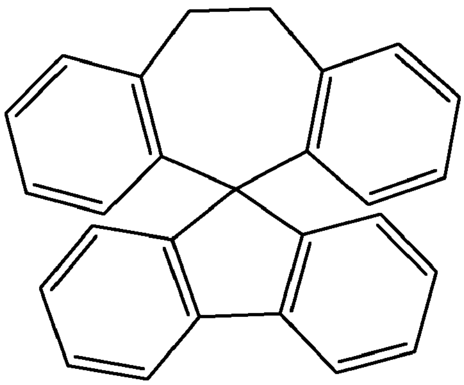

which may be substituted by an amine compound which amine compound comprises a structural element with at least three fused rings with a central ring comprising a nitrogen atom through which the amine is attached to the dibenzosuberane core. One of the non-central fused rings is a non aromatic carbocyclic ring. The general basic structure given for the amine compound is

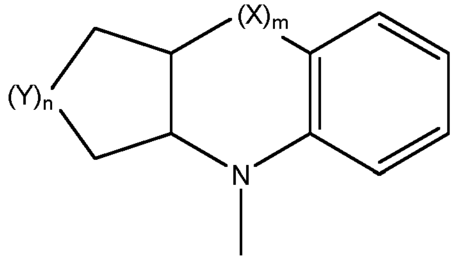

and the attachment to the dibenzosuberane core is through the nitrogen atom.

Japanese Patent Application JP2010/024149 discloses compound having a core element as in KR2012047038 and attached thereto substituents comprising one or more aromatic or non aromatic rings. The attachment to the core is always through a carbon atom of the substituent. Compounds 163 and 167 of this reference show a dibenzosuberane core as in KR2012047038 with two substituted carbazole groups attached to said core through carbon atoms in the phenyl rings of the carbazolyl group.

The compounds known from the prior art are not fully satisfactory as far as electron-transport properties, energy gap and triplet energy is concerned and thus it was an object of the present invention to provide suitable materials of this type with improved properties.

This object is achieved with the compounds in accordance with claim 1.

Preferred embodiments of the present invention are set forth in the dependent claims and in the detailed description hereinafter.

The compounds in accordance with the present invention are characterized by general formula (1)

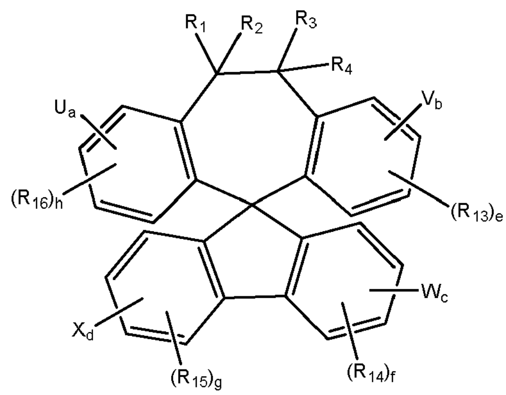

(1)

wherein U, V, W and X, which may be the same or different at each occurrence, represent a substituent group selected from groups of formulas (2) to (4)

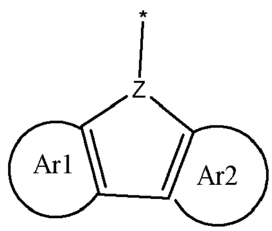

(2)

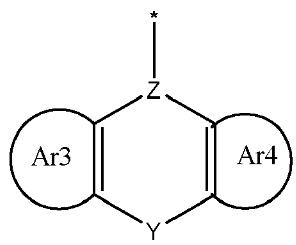

(3)

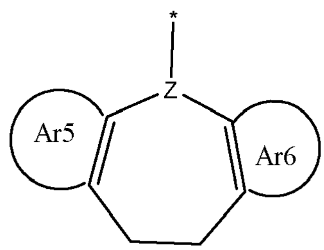

(4)

wherein

Z is N or P,

Y is $CR_5R_6$, $NR_7$, $SiR_8R_9$, $PR_{10}$, $P(=O)R_{11}$ or $BR_{12}$,

$R_5$ to $R_{12}$, which may be the same or different at each occurrence, represent hydrogen or a $C_1$-$C_8$ alkyl group, which may be substituted or unsubstituted and wherein one or more $CH_2$ groups may be replaced by a heteroatom selected from O, N or S and wherein one or more hydrogen atoms may be replaced by another substituent, and

Ar1 to Ar6, which may be the same or different at each occurrence, represents a substituted or unsubstituted five or six membered aryl ring, which may be fused with other rings,

a, b. c and d independently are 0, 1, 2 or 3, preferably o, 1 or 2, more preferably 0 or 1

the sum of a+b+c+d is at least 1,

$R_1$ to $R_4$, which may be the same or different at each occurrence, are selected from H or halogen, cyano, hydroxyl, alkyl, alkenyl, alkynyl, heteroalkyl, heteroalkenyl, heteroalkynyl and alkoxyl with 1 to 18 carbon atoms or $R_1$ and one of $R_3$ and $R_4$ form a chemical bond,

$R_{13}$, $R_{14}$, $R_{15}$ and $R_{16}$, which may be the same or different at each occurrence, are halogen, cyano, hydroxyl, alkoxy, alkyl, alkenyl, alkynyl, heteroalkyl, heteroalkenyl, heteroalkynyl, aryl, heterocyclyl, and heteroaryl and

e, f, g and h are each 0, 1, 2 or 3.

Preferred compounds of the present invention are those wherein at least one of U, V, W and X is represented by formula (2) or formula (3) or formula (4).

In another group of preferred compounds, a and b are 0, i.e no substituents U and V are present.

In still another preferred embodiment $R_1$ to $R_4$ are hydrogen.

e, f, g and h are each, independent of one another, 0, 1, 2 or 3, preferably 0, 1 or 2, more preferably 0 or 1 and, in accordance with a particularly preferred embodiment, e, f, g and h are each 0.

Z is preferably nitrogen.

Ar1 to Ar6, independent of one another are preferably substituted or unsubstituted phenyl.

In accordance with still another embodiment, U, V, W and X, which may be the same or different at each occurrence, are selected from the following carbazole groups

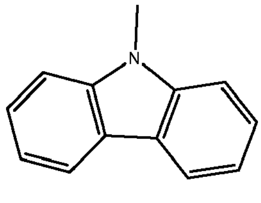
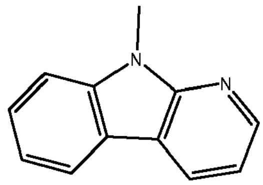
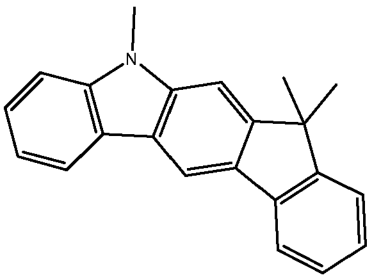
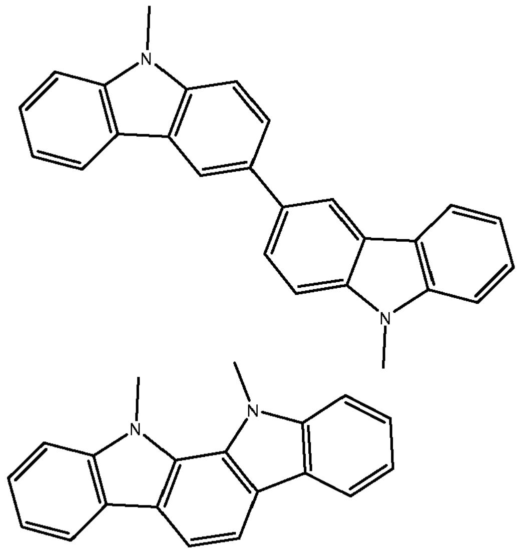
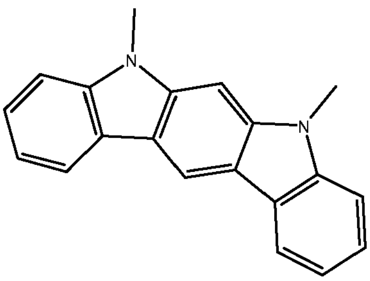
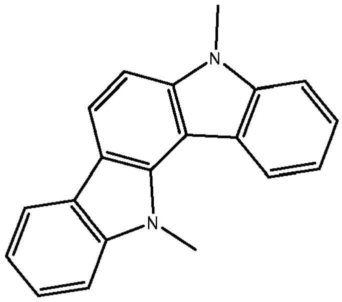
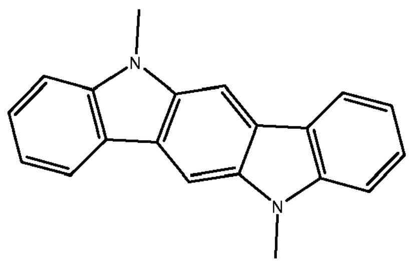

wherein the aromatic rings of the carbazole core may be substituted by one or more substituent or substituents selected from the group consisting of halogen, hydroxy, alkoxy, alkyl, alkenyl or alkinyl, heteroalkyl, heteroalkenyl, heteroalkynyl, aryl, heterocyclyl, and heteroaryl, preferably with an alkyl, alkenyl or alkinyl group, halogen or an alkoxy group. Most preferably, the aromatic rings are unsubstituted and wherein R is selected from the group consisting of halogen, hydroxy, alkoxy, alkyl, alkenyl or alkinyl, heteroalkyl, heteroalkenyl, heteroalkynyl, aryl, heterocyclyl, and heteroaryl, preferably from an alkyl, alkenyl or alkinyl group, halogen or an alkoxy group.

In accordance with still another preferred embodiment, U, V, W and X, which may be the same or different at

each occurrence, are selected from the following groups

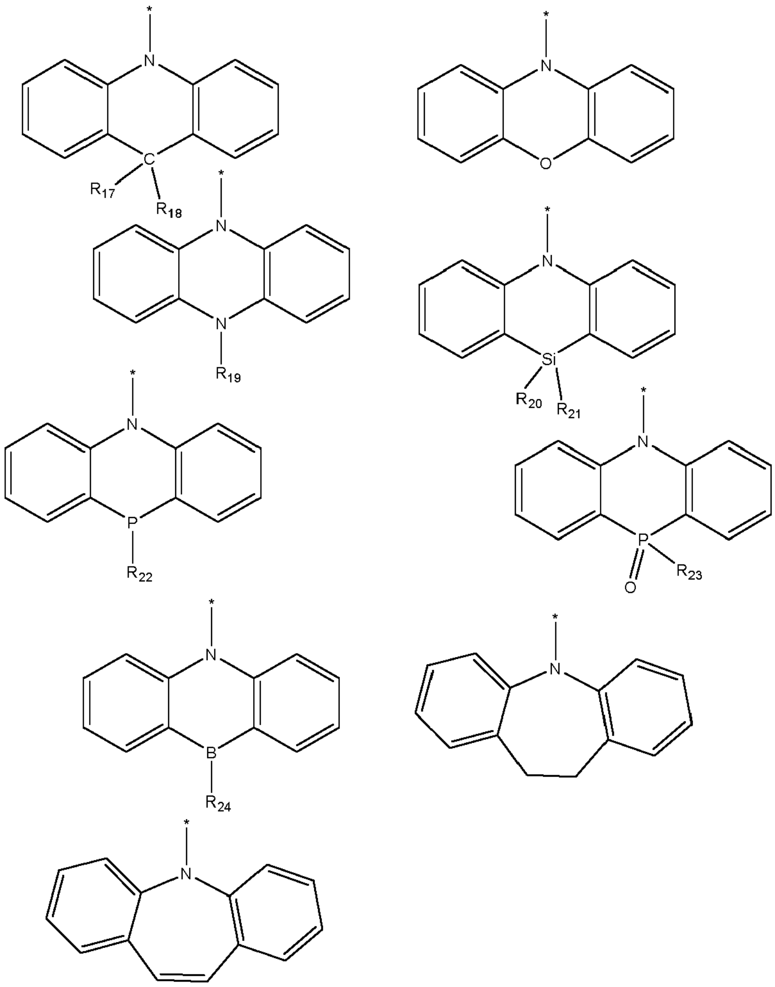

wherein $R_{17}$ to $R_{24}$, which may be the same or different are selected from H, halogen, cyano, hydroxyl and alkyl, alkenyl, alkynyl, heteroalkyl, heteroalkenyl, heteroalkynyl and alkoxyl with 1 to 18 carbon atoms and wherein the aromatic rings may be substituted by one or more substituent or substituents selected from the group consisting of halogen, hydroxy, alkoxy, alkyl, alkenyl or alkinyl, heteroalkyl, heteroalkenyl, heteroalkynyl, aryl, heterocyclyl, and heteroaryl, preferably with an alkyl, alkenyl or alkinyl group, halogen or an alkoxy group. Most preferably, the aromatic rings are unsubstituted.

In accordance with a still further preferred embodiment, the compounds of formula (1) are represented by formulas 1', 1" 1"' and 1""

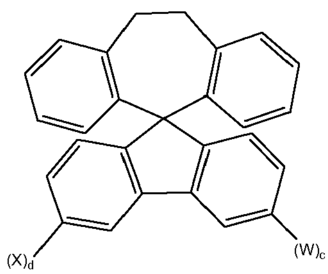

(1')

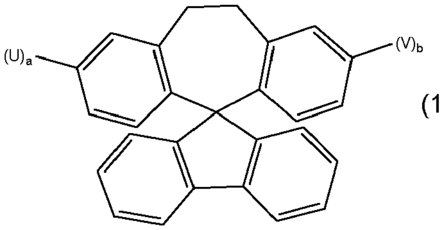

(1'')

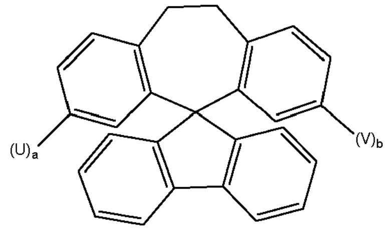

(1''')

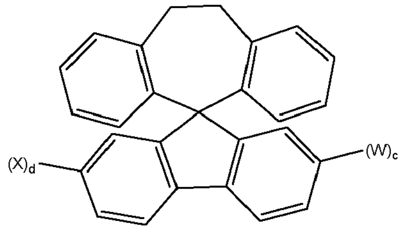

(1'''')

wherein a, b, c and d are each 0, 1, 2 or 3 and the sum of a+b+c+d is 1, 2, 3 or 4, preferably 1 or 2, most preferably 1.

Just by way of example, the following compounds 1-1 to 1-5 may be mentioned as particularly preferred compounds in accordance with the present invention.

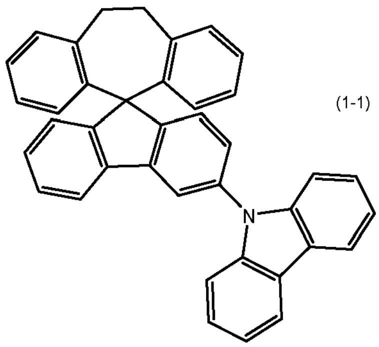

(1-1)

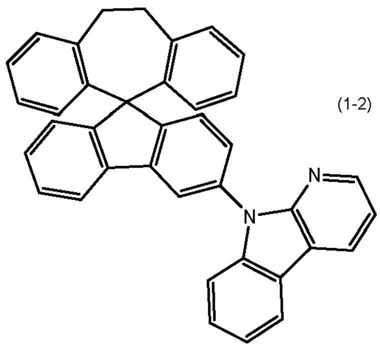

(1-2)

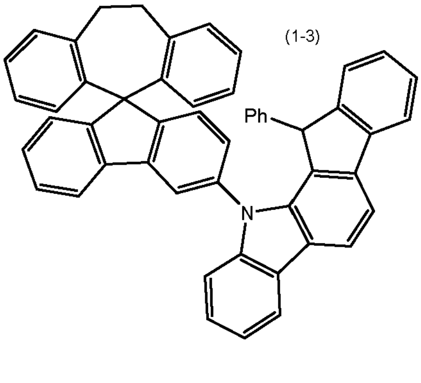

(1-3)

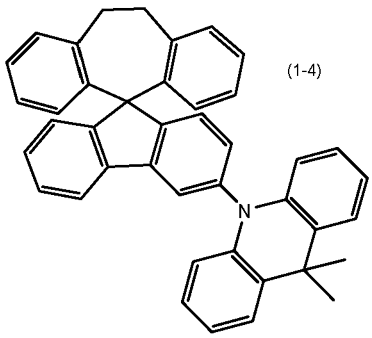

(1-4)

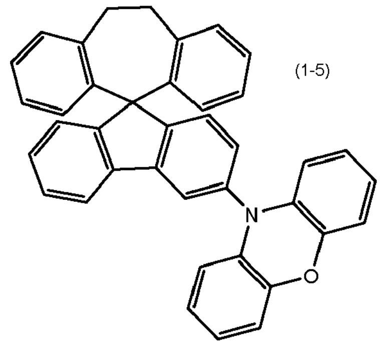

(1-5)

As used herein, the term "halogen" means a halide radical selected from the group consisting of fluoride, chloride, bromide and iodide.

As used herein, the term "alkyl" means a monovalent straight, branched or cyclic saturated hydrocarbon radical, more typically, a monovalent straight or branched saturated ($C_1$-$C_{40}$) hydrocarbon radical, such as, for example, methyl, ethyl, n-propyl, isopropyl, n-butyl, isobutyl, tert-butyl, hexyl, octyl, hexadecyl, octadecyl, eicosyl, behenyl, tricontyl, and tetracontyl.

As used herein, the term "alkoxy" means an alkyl group bonded to an oxygen atom, i.e. a group OR where R denotes an alkyl group as herein defined.

As used herein, the term "cycloalkyl" means a saturated hydrocarbon radical, more typically a saturated ($C_5$-$C_{22}$) hydrocarbon radical, that includes one or more cyclic alkyl rings, which may optionally be substituted on one or more carbon atoms of the ring with one or two ($C_1$-$C_6$)alkyl groups per carbon atom, such as, for example, cyclopentyl, cycloheptyl and cyclooctyl.

As used herein, the term "alkenyl" means an unsaturated straight or branched ($C_2$-$C_{40}$) hydrocarbon radical, more typically an unsaturated straight, branched, ($C_2$-$C_{22}$) hydrocarbon radical, that contains one or more carbon-carbon double bonds, including, for example, ethenyl (vinyl), n-propenyl, and iso-propenyl, and allyl.

As used herein, the term "alkynyl" means an unsaturated straight or branched ($C_2$-$C_{40}$) hydrocarbon radical, more typically an unsaturated straight, branched, ($C_2$-$C_{22}$) hydrocarbon radical, that contains one or more carbon-carbon triple bonds, including, for example, ethynyl, propynyl, and butynyl.

The term "heteroalkyl" means an alkyl group wherein one or more of the carbon atoms within the alkyl group has been replaced by a hetero atom, such as, for example, nitrogen, oxygen, or sulfur.

The term "heteroalkenyl" means an alkenyl group wherein one or more of the carbon atoms within the alkenyl group has been replaced by a hetero atom, such as, for example, nitrogen, oxygen, or sulfur.

The term "heteroalkynyl" means an alkynyl group wherein one or more of the carbon atoms within the alkynyl group has been replaced by a hetero atom, such as, for example, nitrogen, oxygen, or sulfur.

As used herein, the term "aryl" means a monovalent unsaturated hydrocarbon radical containing one or more carbon rings having a total number of $\pi$-electrons following the so called Hückel rule, i.e. having $4n+2$-$\pi$-electrons with n being an integer of at least 1. Aryl radicals include monocyclic aryl and polycyclic aryl. "Polycyclic aryl" refers to a monovalent unsaturated hydrocarbon radical containing more than one six-membered carbon ring in which the unsaturation may be represented by three conjugated double bonds wherein adjacent rings may be linked to each other by one or more bonds or divalent bridging groups or may be fused together. Aryl radicals may be substituted at one or more carbons of the ring or rings with any substituent described herein. Examples of aryl radicals include, but are not limited to, phenyl, methylphenyl, isopropylphenyl, tert-butylphenyl, methoxyphenyl, dimethylphenyl, trimethylphenyl, chlorophenyl, trichloromethylphenyl, triisobutyl phenyl, anthracenyl, naphthyl, phenanthrenyl, fluorenyl, and pyrenyl.

As used herein, the term "heterocycle" or "heterocyclic" refers to compounds having a saturated or partially unsaturated cyclic ring structure that includes one or more hetero atoms in the ring. The term "heterocyclyl" refers to a monovalent group having a saturated or partially unsaturated cyclic ring structure that includes one or more hetero atoms in the ring. Examples of heterocyclyl groups include, but are not limited to, morpholinyl, piperadinyl, piperazinyl, pyrrolinyl, pyrazolyl, and pyrrolidinyl.

As used herein, the term "heteroaryl" means a monovalent group having at least one aromatic ring that includes at least one hetero atom in the ring, which may be substituted at one or more atoms of the ring with hydroxyl, alkyl, alkoxyl, alkenyl, halogen, haloalkyl, monocyclic aryl, or amino. Examples of heteroaryl groups include, but are not limited to, thienyl, pyrrolyl, pyridinyl, pyrimidinyl, pyrazinyl, triazinyl, pyridazinyl, tetrazolyl, and imidazolyl groups. The term "polycyclic heteroaryl" refers to a monovalent group having more than one aromatic ring, at least one of which includes at least one hetero atom in the ring, wherein adjacent rings may be linked to each other by one or more bonds or divalent bridging groups or may be fused together. Examples of polycyclic heteroaryl groups include, but are not limited to, indolyl

and quinolinyl groups.

The compounds in accordance with the present invention may be obtained in accordance with processes known per se and the skilled person will select the appropriate reaction pathway tailored to the target compound of formula (1). A suitable reaction pathway is e.g. described in KR 2012/0047038 starting on page 82, to which reference is made herewith for further details.

A preferred process for the manufacture of the compounds of the present invention is now described. In a first reaction step, a halogenated phenyl derivative is coupled with a halogenated boronic acid derivative in the presence of a palladium catalyst. The halogen atoms include chlorine, bromine and iodine. A solvent capable of dissolving the raw materials may be used for the reaction for example, toluene, xylene, tetrahydrofuran, dioxane, tetralin, quinolone, nitrobenzene, dimethyl sulfoxide and N,N-dimethylformamide.

The aforementioned coupling reaction can be performed in the presence of a metal catalyst and a base at a temperature in the range of 60-200°C. Example of the catalyst include a palladium complex that is formed from a palladium source such as palladium actetate and bis(dibenzylideneacetone) palladium and a ligand such as triphenylphosphine or tri-tert-butylphosphine. Example of the base include inorganic bases such as potassium carbonate, sodium carbonate, cesium carbonate, sodium hydroxide, sodium tert-butoxide, potassium tert-butoxide and organic bases such as pyridine, picoline or triethylamine.

The second reaction involved to obtain the compound of this invention is a lithium-halogen exchange reaction between the halogenated derivative obtained following the coupling reaction in the presence of an alkyl lithium compound. The halogen atoms include chlorine, bromine and iodine. The alkyl lithium compound used for the reaction can e.g. be tert-butyl lithium, sec-butyl lithium or n-butyl lithium. The solvent used for the reaction can be e.g. tetrahydrofuran, diethyl ether or hexane. The lithium-halogen exchange reaction can be performed e.g. at a temperature in the range of -100°C to +30°C.

The lithiated derivative obtained in the aforementioned reaction is reacted with dibenzosuberone in an appropriate solvent among which tetrahydrofuran, diethyl ether, hexane. Upon completion of the reaction water is added to the reaction mixture to separate an organic layer, and the organic layer is concentrated, washed with a low boiling solvent such as ethyl acetate or dichloromethane and dried under reduced pressure to give the intermediate alcohol to the compound of this invention.

The compounds of the present invention are then obtained at last in a cyclization reaction in presence of a catalytic amount of a strong acid among which trifluoromethane sulfonic acid, trifluoroacetic acid or nitric acid may be mentioned. The solvent used for the reaction can be a low boiling solvent as for example dichloromethane.

The overall reaction scheme can be summarized as follows:

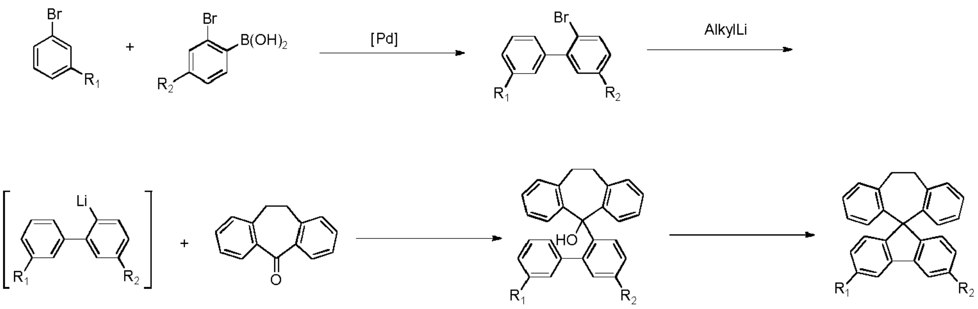


The compounds of the present invention may be advantageously used for the formation of layers in organic electronic devices, in particular in light emitting diodes, field-effect transistors or in photovoltaic cells.

Accordingly, a further object of the present invention are devices comprising one or more than one layers comprising at least one compound in accordance with the present invention.

In accordance with a particularly preferred embodiment, the compounds of the present invention are used as host in the emissive layer of an organic light emitting diode or as charge transport material in the electron transporting and/or the and exciton blocking layer in an organic light emitting diode.

An OLED generally comprises :

a substrate, for example (but not limited to) glass, plastic, metal;
an anode, generally transparent anode, such as an indium-tin oxide (ITO) anode;
a hole injection layer (HIL) for example (but not limited to) PEDOT/PSS;
a hole transporting layer (HTL);
an emissive layer (EML);
an electron transporting layer (ETL);

an electron injection layer (EIL) such as LiF, $Cs_2CO_3$
a cathode, generally a metallic cathode, such as an Al layer.

For a hole conducting emissive layer, one may have a hole blocking layer (HBL) that can also act as an exciton blocking layer between the emissive layer and the electron transporting layer. For an electron conducting emissive layer, one may have an electron blocking layer (EBL) that can also act as an exciton blocking layer between the emissive layer and the hole transporting layer. The emissive layer may be equal to the hole transporting layer (in which case the exciton blocking layer is near or at the anode) or to the electron transporting layer (in which case the exciton blocking layer is near or at the cathode).
The emissive layer may be formed with a host material in which the light emitting material or mixture of these materials as above described resides as a guest or the emissive layer may consist essentially of the light emitting material or mixture of these materials as above described itself.
Optionally, the emissive layer may also contain a polarization molecule, present as a dopant in said host material and having a dipole moment, that generally affects the wavelength of light emitted when said light emitting material as above described, used as a dopant, luminesces.
A layer formed of an electron transporting material is advantageously used to transport electrons into the emissive layer comprising the light emitting material and the (optional) host material.
A layer formed of a hole transporting material is advantageously used to transport holes into the emissive layer comprising the light emitting material as above described and the (optional) host material.
The use of an exciton blocking layer ("barrier layer") to confine excitons within the luminescent layer ("luminescent zone") is greatly preferred. For a hole-transporting host, the blocking layer may be placed between the emissive layer and the electron transport layer.
The compounds in accordance with the present invention in preferred embodiments show a good solubility in organic solvents thereby favoring processability from solution which is advantageous for low cost OLED production.
They may be formulated in a suitable solvent together with a liquid carrier as an ink composition which may be printed by suitable equipment.
The photophysical, electrochemical, and thermal properties of the compounds of the present invention can be characterized using standard methods and apparatuses known to those of ordinary skill in the art. Ultraviolet-visible (UV-Vis) spectra may be obtained with a spectrophotometer, such as, for example, Perkin-Elmer model Lambda 900 UV/vis/near-IR spectrophotometer. Photoluminescence (PL) spectra may be obtained using a spectrofluorimeter, such as, for example, a Photon Technology International (PTI) Inc. Model QM 2001-4 spectrofluorimeter.
The following example shows a preferred embodiment of the present invention.

Example 1 - Synthesis of compound 1-1

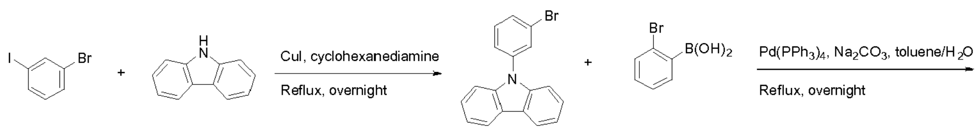


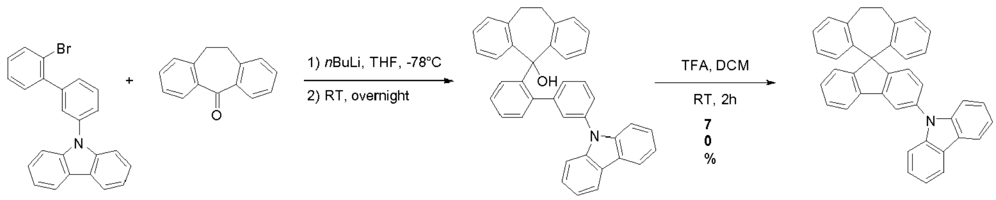


I II III

Compound **I** (5.01 g, 12.6 mmol, synthesized e.g. as described in Chem. Comm. 2013, 49, 9860-9862; J. Chem. Mater. 2011, 23, 274 and Dyes Pigments 2014, 101, 150) was dissolved in THF (80 mL) and the resulting mixture was cooled down to -78°C. nBuLi 1.6M in hexane (9.5 mL, 15.2 mmol) was subsequently added to the solution and left to react for 2h at this temperature. A solution of dibenzosuberone (2.09 g, 10 mmol) in THF (50 mL) was slowly added and the temperature was raised to room temperature. After 4h, the reaction mixture was quenched with water and the solvents evaporated. The residue was extracted with dichloromethane (3 x 20 mL) and washed with three times with water (3 x 20 mL). The organic phase was dried with $MgSO_4$ and concentrated *in vacuo.* The product was washed several times

with hexane, dried and used directly without further purification in the following step.

Compound **II** was dissolved in dichloromethane (30 mL) and trifluoroacetic acid (7.5 mL, 44 mmol) was added dropwise. After 2h at room temperature, the resulting solution was quenched with water and the organic phase washed with a 1 M solution of $Na_2CO_3$ until the water phase had reached pH=7. The organic phase was then washed with water, dried with $MgSO_4$ and concentrated *in vacuo.* The residue was purified by flash chromatography using a mixture of hexane/ethyl acetate as the eluent to yield the pure product as a white solid (4.2 g, 82% yield).

$^1$H NMR (500 MHz, THF-$d_8$) $\delta$ = 8.29 (d, 2H, $J_{HH}$ = 7.5 Hz; Ar*H*), 8.08 (s, 1H; Ar*H*), 7.88 (d, 1H, $J_{HH}$ = 8 Hz; Ar*H*), 7.62-7.53 (m, 4H; Ar*H*), 7.47-7.41 (m, 5H; Ar*H*), 7.36-7.28 (m, 4H; Ar*H*), 7.18 (t, 2H, $J_{HH}$ = 7 Hz; Ar*H*), 6.99 (t, 2H, $J_{HH}$ = 7.5 Hz; Ar*H*), 6.64 (d, 2H, $J_{HH}$ = 8.5 Hz; Ar*H*), 3.55 (s, 4H; C$H_2$).

The molecule was fully characterized to determine its optical and electronic properties and the data collected in the table below. The features of the new material are comparable with those of the spirobifluorene-based corresponding compound, exhibiting a high triplet energy and a slightly shallower HOMO level.

The properties of the compound are given in Table 1:

Table 1

| $E_T$ | $E_{OptG}$ | HOMO | LUMO | $T_{decomp}$ | $M_W$ |
|---|---|---|---|---|---|
| 2.84 | 3.47 | -5.66 eV | -2.19 eV | 1%@313°C | 509.6 |

The triplet energy was calculated from the highest energy phosphorescence peak measured in 2-Me-THF at 77 K. (The photoluminescence measurements were performed on highly diluted (= $10^{-5}$ mol/L) solutions in spectroscopic grade of 2-methyl-THF using a HORIBA JOBIN YVON Fluoromax-4 P spectrofluorimeter. Measurements at 77 K were carried out using the FL-2013 Dewar liquid nitrogen assembly from HORIBA JOBIN YVON.

EoptG (ev) was measured from the onset wavelength determined by adsorption spectroscopy

The HOMO and LUMO levels of the organic molecules used in the process of the present invention are determined from cyclic voltammetry measurements in solution as follows:

The measurements are performed at room temperature, under inert atmosphere, with a conventional three-electrode configuration, the solution being outgassed before use with a stream of argon for 5 -10 min. The three-electrode cell may consist e.g. of a glassy carbon disk as working electrode, a Pt wire or a Pt rod as a counter electrode and a Pt wire or a carbon rod as pseudo-reference electrode. Ferrocene is used as an internal reference. Other cell configurations may also be used. The solvents used for the determination of the HOMO and LUMO levels are respectively anhydrous dichloromethane and anhydrous tetrahydrofuran, the supporting electrolyte is 0.1 M tetrabutylammonium hexafluorophosphate and the host concentrations are 2 - 0.5 millimolar. The scan rate is fixed to 100 mv/s.

The HOMO levels ($E_{HOMO}$) are calculated from the measured half wave potential of their first oxidation wave ($E_{1\ ox\ 1/2}$) using the following equation:

$$E_{HOMO} - (-4.8) = -[E_{1\ ox\ 1/2} - E_{ox\ 1/2}(Fc/Fc^+)]$$

wherein the ferrocene HOMO level value has been taken equal to -4.8 eV below the vacuum level according to Pommerehene and al. Adv. Mater. 7(6), 551-554 (1995) and wherein $E_{ox\ 1/2}(Fc/Fc^+)$ corresponds to the measured half wave potential of the ferrocene oxidation wave. For irreversible systems, $E_{pa\ 1}$ peak potential value of the first oxidation wave is used instead of the half wave potential $E_{1ox\ 1/2}$.

The LUMO levels ($E_{LUMO}$) are calculated from the measured half wave potential of their first reduction wave ($E_{1ox\ 1/2}$) using the following equation:

$$E_{LUMO} - (-4.8) = -[E_{1\ red\ 1/2} - E_{ox\ 1/2}(Fc/Fc^+)]$$

wherein the ferrocene HOMO level value has been taken equal to -4.8 eV below the vacuum level according to Pommerehene et al. Adv. Mater. 7(6), 551-554 (1995) and wherein $E_{ox\ 1/2}(Fc/Fc^+)$ corresponds to the measured half wave potential of the ferrocene oxidation wave. For irreversible systems, $E_{pc\ 1}$ peak potential value of the first reduction wave is used instead of the half wave potential $E_{1\ red\ ½}$.

The decomposition temperature was determined at the temperature where a weight loss of 1 % is observed upon heating a sample of the compound at a heating rate of 10 K/min.

## Claims

1. Compounds of formula

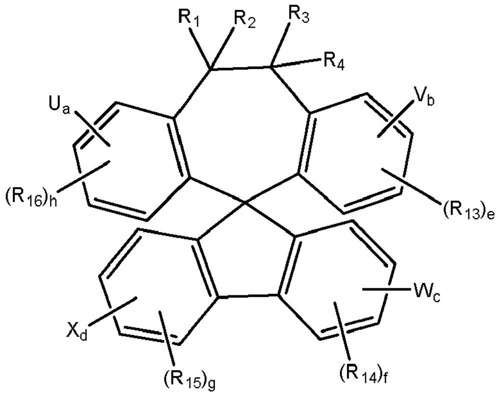

(1)

wherein U, V, W and X, which may be the same or different at each occurrence, represent a substituent group selected from

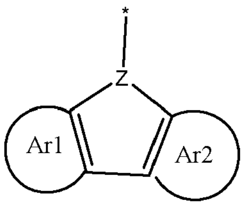

(2)

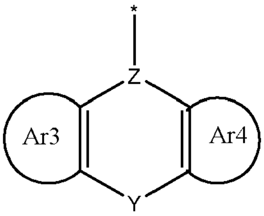

(3)

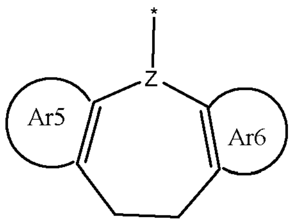

(4)

wherein
Z is N or P,
Y is $CR_5R_6$, $NR_7$, $SiR_8R_9$, $PR_{10}$, $P(=O)R_{11}$ or $BR_{12}$,
$R_5$ to $R_{12}$, which may be the same or different at each occurrence, represent hydrogen or a $C_1$-$C_8$ alkyl group, which may be substituted or unsubstituted and wherein one or more $CH_2$ groups may be replaced by a heteroatom selected from O, N or S and wherein one or more hydrogen atoms may be replaced by another substituent, and
Ar1 to Ar6, which may be the same or different at each occurrence, represents a substituted or unsubstituted five or six membered aryl ring, which may be fused with other rings,
a, b. c and d independently are 0, 1, 2 or 3,
the sum of a+b+c+d is at least 1,
$R_1$ to $R_4$, which may be the same or different, are selected from H or halogen, cyano, hydroxyl, alkyl, alkenyl, alkynyl, heteroalkyl, heteroalkenyl, heteroalkynyl and alkoxyl with 1 to 18 carbon atoms or $R_1$ and one of $R_3$ and $R_4$ form a chemical bond,
$R_{13}$, $R_{14}$, $R_{15}$ and $R_{16}$, which may be the same or different at each occurrence, are halogen, cyano, hydroxyl, alkoxy, alkyl, alkenyl, alkynyl, heteroalkyl, heteroalkenyl, heteroalkynyl, aryl, heterocyclyl, and heteroaryl and
e, f, g and h are each 0, 1, 2 or 3.

2. Compounds in accordance with claim 1 wherein at least one of U, V, W and X is represented by formula (2).

3. Compounds in accordance with claim 1 wherein at least of U, V, W and X is represented by formula (3).

4. Compounds in accordance with claim 1 wherein at least one of U, V, W and X is represented by formula (4).

5. Compounds in accordance with any of claims 1 to 4 wherein a and b are 0.

6. Compounds in accordance with claim any of claims 1 to 5 wherein $R_1$ to $R_4$ are hydrogen.

7. Compounds in accordance with any of claims 1 to 6 wherein Z is nitrogen.

8. Compounds in accordance with any of claims 1 to 7 wherein Ar1 to Ar6, which may be the same or different, are substituted or unsubstituted phenyl.

9. Compounds in accordance with any of claims 1, 2 and 5 to 8 wherein U, V, W and X are selected from the following carbazole groups

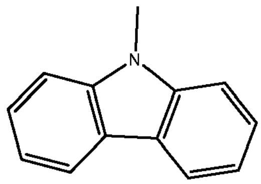

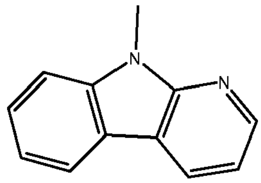

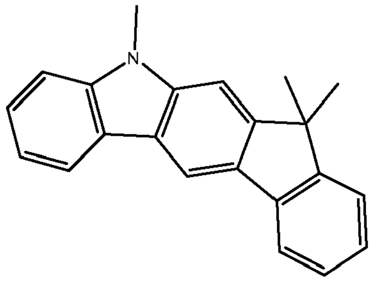

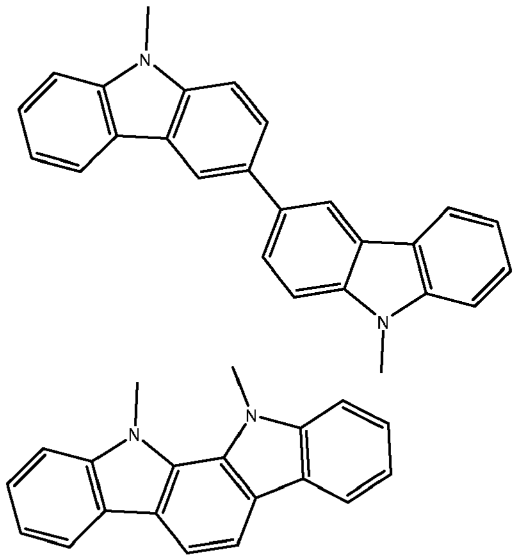

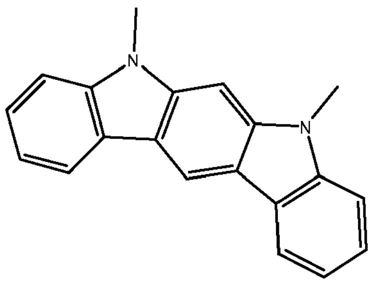

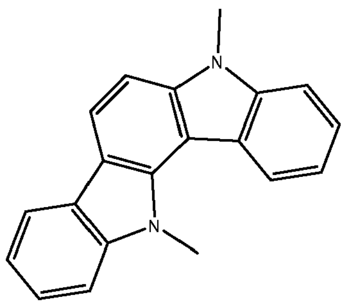

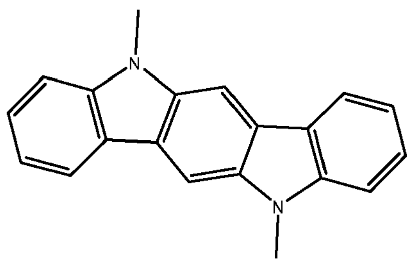

wherein the aromatic rings of the carbazole core may be substituted by one or more substituent or substituents selected from the group consisting of halogen, hydroxy, alkoxy, alkyl, alkenyl or alkinyl, heteroalkyl, heteroalkenyl, heteroalkynyl, aryl, heterocyclyl, and heteroaryl.

10. Compounds in accordance with any of claims 1 and 3 to 8 wherein U, V, W and X are selected from the following groups:

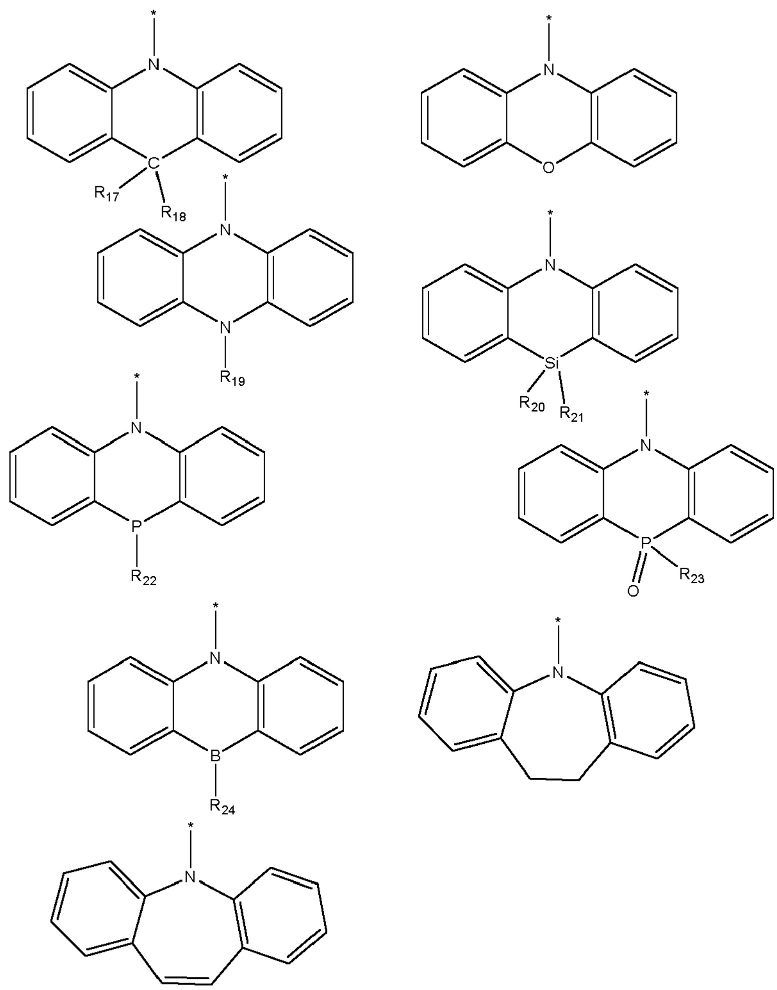

wherein $R_{17}$ to $R_{24}$, which may be the same or different are selected from H, halogen, cyano, hydroxyl and alkyl, alkenyl, alkynyl, heteroalkyl, heteroalkenyl, heteroalkynyl and alkoxyl with 1 to 18 carbon atoms and wherein the aromatic rings may be substituted by one or more substituent or substituents selected from the group consisting of halogen, hydroxy, alkoxy, alkyl, alkenyl or alkinyl, heteroalkyl, heteroalkenyl, heteroalkynyl, aryl, heterocyclyl, and heteroaryl.

**11.** A composition comprising at least one compound according to any one of claims 1 to 10.

**12.** An ink composition comprising at least one liquid carrier and at least one compound according to any one of claims 1 to 10.

**13.** A device comprising one or more than one layers comprising at least one compound according to at least one of claim 1-10.

**14.** The device of claim 14 which is a light emitting diode, a field-effect transistor or a photovoltaic cell.

**15.** Use of the compounds in accordance with any one of claims 1 to 10 in the electron transporting and/or hole and exciton blocking layer in an organic light emitting diode.

Europäisches Patentamt
European Patent Office
Office européen des brevets

# EUROPEAN SEARCH REPORT

Application Number
EP 14 19 9901

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WEI-SHAN CHAO ET AL: "Supporting Information Spirally Configured cis-stilbene/Fluorene Hybrids as Bipolar, Organic Sensitizers for Solar Cell Applications", CHEMICAL COMMUNICATIONS, vol. 48, 1 January 2012 (2012-01-01), pages S1-s34, XP055167702, ISSN: 1359-7345, DOI: 10.1039/c2cc17079e * page S14, paragraph Electrode preparation and device fabrication - page S16, paragraph; compounds IIe, IIIe, dye 5 * | 1,4,7,8, 10-14 | INV. C07D209/86 H01L51/52 |
| X | WEI-SHAN CHAO ET AL: "Spirally configured cis-stilbene/fluorene hybrids as bipolar, organic sensitizers for solar cell applications", CHEMICAL COMMUNICATIONS, vol. 48, no. 40, 1 January 2012 (2012-01-01), page 4884, XP055167667, ISSN: 1359-7345, DOI: 10.1039/c2cc17079e * the whole document * | 1,4,7,8, 10,11, 13,14 | |
| X,D | JP 2010 024149 A (TOYO INK MFG CO) 4 February 2010 (2010-02-04) * Formula [3]; claims 1-13; compounds 167, 168, 180 * | 1-15 | TECHNICAL FIELDS SEARCHED (IPC) C07D H01L |
| X | US 2014/312311 A1 (CHEN CHIEN-TIEN [TW] ET AL) 23 October 2014 (2014-10-23) * paragraph [0029] - paragraph [0038]; claims 1-6 * | 1-15 | |
| X | KR 2012 0047038 A (SFC CO LTD [KR]) 11 May 2012 (2012-05-11) * compounds 141, 185-187, 191-202 * | 1-15 | |

The present search report has been drawn up for all claims

2

| Place of search | Date of completion of the search | Examiner |
|---|---|---|
| Munich | 10 February 2015 | Sotoca Usina, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 19 9901

This annex lists the patent family members relating to the patent documents cited in the above-mentioned European search report. The members are as contained in the European Patent Office EDP file on
The European Patent Office is in no way liable for these particulars which are merely given for the purpose of information.

10-02-2015

| Patent document cited in search report | Publication date | Patent family member(s) | Publication date |
|---|---|---|---|
| JP 2010024149 A | 04-02-2010 | NONE | |
| US 2014312311 A1 | 23-10-2014 | CN 104109105 A | 22-10-2014 |
| | | TW 201441182 A | 01-11-2014 |
| | | US 2014312311 A1 | 23-10-2014 |
| KR 20120047038 A | 11-05-2012 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 2012047038
- JP 2010024149 A
- KR 20120047038


### Non-patent literature cited in the description


- *Chem. Comm.,* 2013, vol. 49, 9860-9862
- *J. Chem. Mater.,* 2011, vol. 23, 274
- *Dyes Pigments,* 2014, vol. 101, 150
- **POMMEREHENE.** *Adv. Mater.,* 1995, vol. 7 (6), 551-554
- **POMMEREHENE et al.** *Adv. Mater.,* 1995, vol. 7 (6), 551-554